# EUROPEAN PATENT APPLICATION

(11) **EP 1 635 160 A2**
(43) Date of publication of application: **15.03.2006**
(21) Application number: 05255529.9
(22) Date of filing: 08.09.2005
(51) Int. Cl.: G01N 15/12, G12B 21/20, B81B 5/00, C12Q 1/68, G01N 33/483

(54) **Nanostepper/sensor systems and methods of use thereof**

(30) Priority: 10.09.2004 US 938213
(71) Applicant: Agilent Technologies, Inc., Palo Alto, CA 94306 (US)
(72) Inventor: McAllister, William H., Saratoga, CA 95070 (US)
(74) Representative: Exell, Jonathan Mark

(57) **Abstract**

A system and method are disclosed including a nanopore system (30a) including a structure having a nanopore aperture (34); and a first nanostepper system (20) having an x-/y-direction moving structure (22) and a first nanostepper arm (24) positioned adjacent the structure (32), wherein the first nanostepper arm (24) is adapted to interact with a target polymer (38), wherein the x-/y-direction moving structure (22) is operative to position the first nanostepper arm (24) having the target polymer (38) disposed thereon substantially inline with the nanopore aperture (34), and wherein the x-/y-direction moving structure (22) is operative to controllably translocate the target polymer (38) through the nanopore aperture (34).

## Description

Determining the nucleotide sequence of DNA and RNA in a rapid manner is a major goal of researchers in biotechnology, especially for projects seeking to obtain the sequence of entire genomes of organisms. In addition, rapidly determining the sequence of a nucleic acid molecule is important for identifying genetic mutations and polymorphisms in individuals and populations of individuals.

Nanopore sequencing is one method of rapidly determining the sequence of nucleic acid molecules. Nanopore sequencing is based on the property of physically sensing the individual nucleotides (or physical changes in the environment of the nucleotides (*i.e*., electric current)) within an individual polynucleotide *(e.g.,* DNA and RNA) as it traverses through a nanopore aperture. In principle, the sequence of a polynucleotide can be determined from a single molecule. However, in practice, it is preferred that a polynucleotide sequence be determined from a statistical average of data obtained from multiple passages of the same molecule or the passage of multiple molecules having the same polynucleotide sequence. The use of membrane channels to characterize polynucleotides as the molecules pass through the small ion channels has been studied by Kasianowicz *et al.* (*Proc. Natl. Acad. Sci.,* USA. 93:13770-3, 1996, incorporated herein by reference) by using an electric field to force single stranded RNA and DNA molecules through a 2.6 nanometer diameter nanopore aperture (i.e., ion channel) in a lipid bilayer membrane. The diameter of the nanopore aperture permitted only a single strand of a polynucleotide to traverse the nanopore aperture at any given time. As the polynucleotide traversed the nanopore aperture, the polynucleotide partially blocked the nanopore aperture, resulting in a transient decrease of ionic current. Since the length of the decrease in current is directly proportional to the length of the polynucleotide, Kasianowicz *et al.* were able to experimentally determine lengths of polynucleotides by measuring changes in the ionic current.

Baldarelli *et al.* (U.S. Pat. No. 6,015,714) and Church *et al.* (U.S. Pat. No. 5,795,782) describe the use of nanopores to characterize polynucleotides including DNA and RNA molecules on a monomer by monomer basis. In particular, Baldarelli *et al.* characterized and sequenced the polynucleotides by passing a polynucleotide through the nanopore aperture. The nanopore aperture is embedded in a structure or an interface, which separates two media. As the polynucleotide passes through the nanopore aperture, the polynucleotide alters an ionic current by blocking the nanopore aperture. As the individual nucleotides pass through the nanopore aperture, each base/nucleotide alters the ionic current in a manner which allows the identification of the nucleotide transiently blocking the nanopore aperture, thereby allowing one to characterize the nucleotide composition of the polynucleotide and perhaps determine the nucleotide sequence of the polynucleotide.

One disadvantage of previous nanopore analysis techniques is controlling the rate at which the target polynucleotide is analyzed. As described by Kasianowicz *et al.* (*Proc. Natl. Acad. Sci.,* USA, 93:13770-3, (1996)), nanopore analysis is a useful method for performing length determinations of polynucleotides. However, the translocation rate is nucleotide composition dependent and can range between 10⁵ to 10⁷ nucleotides per second under the measurement conditions outlined by Kasianowicz *et al.* Therefore, the correlation between any given polynucleotide's length and its translocation time is not straightforward. It is also anticipated that a higher degree of resolution with regard to both the composition and spatial relationship between nucleotide units within a polynucleotide can be obtained if the translocation rate is substantially better controlled, both in speed and regularity. Another disadvantage of previous nanopore analysis techniques is that each individual polymer typically passes through the detection region only once.

According to an aspect of the present invention, there is provided a method according to claim 1. According to another aspect of the present invention, there is provided a system according to claim 4. According to another aspect of the present invention, there is provided a method according to claim 6. According to another aspect of the present invention, there is provided a system according to claim 7. The present invention seeks to provide nanostepper/sensor systems and methods for analyzing a polymer. In one embodiment, a nanopore system and a first nanostepper system are used. The nanopore system includes a structure having a nanopore aperture. The first nanostepper system includes an x-/y-direction moving structure and a first nanostepper arm positioned adjacent the structure. The first nanostepper arm is adapted to interact with a target polymer, where the x-/y-direction moving structure is operative to position the first nanostepper arm having the target polymer disposed thereon substantially inline with the nanopore aperture. The x-/y-direction moving structure is operative to controllably translocate the target polymer through the nanopore aperture.

In another embodiment, a sensor system and a nanostepper system are used. The sensor system includes a sensor. The nanostepper system includes an x-/y-direction moving structure and a nanostepper arm positioned adjacent the sensor. The nanostepper arm is adapted to interact with a target polymer, wherein the x-/y-direction moving structure is operative to position the nanostepper arm having the target polymer disposed thereon substantially adjacent the sensor. The x-/y-direction moving structure is operative to controllably and reversibly move the target polymer near the sensor such that the sensor senses the target polymer.

In another embodiment, a method for analyzing a polymer includes: translocating a target polymer through a nanopore aperture in a controllable, repeatable, and reversible manner using a first x-/y-direction moving structure, wherein the x-/y-direction moving structure is operative to position the target polymer substantially in-line with the nanopore aperture, wherein the x-/y-direction moving structure is operative to move independently in the x- and y-directions, and wherein the x-direction is defined as an axis through a structure in which the nanopore is formed; and monitoring the signal corresponding to the movement of the target polymer with respect to the nanopore aperture as a function of the movement of the first x-/y-direction moving structure.

In another embodiment, a method includes: moving a target polymer adjacent a sensor in a controllable, repeatable, and reversible manner using a nanostepper system, wherein the nanostepper system is operative to position the target polymer substantially near the sensor, wherein the nanostepper system is operative to move independently in the x- and y-directions, wherein the x-direction is in the same plane as the sensor and the y-direction moves the target polymer to the left and right of the sensor; and monitoring the signal corresponding to the movement of the target polymer with respect to the sensor as a function of the movement of the nanostepper system.

Other systems, methods, features and/or advantages will be or may become apparent to one with skill in the art upon examination of the following drawings and detailed description. It is intended that all such additional systems, methods, features and/or advantages be included within this description and be protected by the accompanying claims.

Embodiments of the present invention will now be described in detail, with reference to the accompanying drawings, in which:
FIG. 1 is a schematic diagram of a nanostepper/sensor system according to an embodiment of the present invention.
FIG. 2 is a flow diagram of a representative process for using the nanostepper/sensor system illustrated in FIG. 1.
FIG. 3 is a diagram of representative nanostepper/sensor system.
FIG. 4 is a flow diagram of a representative process for using the nanostepper/sensor system illustrated in FIG. 3.
FIGS. 5A through 5F are diagrams of a representative process using the nanostepper/sensor system illustrated in FIG. 3.
FIG. 6 is a diagram of a representative nanostepper/sensor system incorporating a pair of nanosteppers.
FIG. 7 is a diagram of a representative nanostepper/sensor system incorporating a flexure system.
FIG. 8 is a diagram of a representative nanostepper/sensor system incorporating two nanosteppers and a notch system.
FIGS. 9A through 9B are diagrams of a representative nanostepper/sensor system incorporating an array system.

As will be described in greater detail herein, nanostepper/sensor systems and methods of use thereof, are provided. The term "nanostepper" refers to a micromachined electrostatic actuator, which is described in greater detail in U.S. Patent No. 5,986,381 and is incorporated herein by reference. The nanostepper uses an electrostatically actuated dipole surface drive that has demonstrated forces of up to several hundred microNewtons while traveling about 50 microns. The dipole stepping motor design allows this device to provide large forces, while traveling long distances along two directions. With active feedback the nanostepper can be repeatably positioned with a precision of about 1.5 nanometers and down to about 1 Angstrom.

By way of example, some embodiments provide for nanostepper/sensor systems having a nanostepper system operative to move a target polymer (e.g., polypeptide and polynucleotide) controllably and reversibly adjacent (e.g., in close proximity) to a sensor such that the sensor senses the target polymer (e.g., detects one or more characteristics of the target polymer). Embodiments of the nanostepper/sensor system provide a robust method for the physical placement and alignment of the target polymer relative to the sensor. In addition, the nanostepper/sensor system is operative to control the rate of movement of the target polymer as it passes the sensor. In this regard, the nanostepper/sensor system can reverse the movement of the target polymer, which enables the target polymer to be sensed (analyzed) by the sensor a plurality of times. Further, the nanostepper/sensor system is operative to exert force on the target polymer to "stretch" the target polymer to be substantially linear from a coiled or nonlinear conformation. A further advantage of using the nanostepper/sensor system is that the probability of backward movement of the target polymer is substantially decreased, thus ensuring a defined directional analysis of the target polymer.

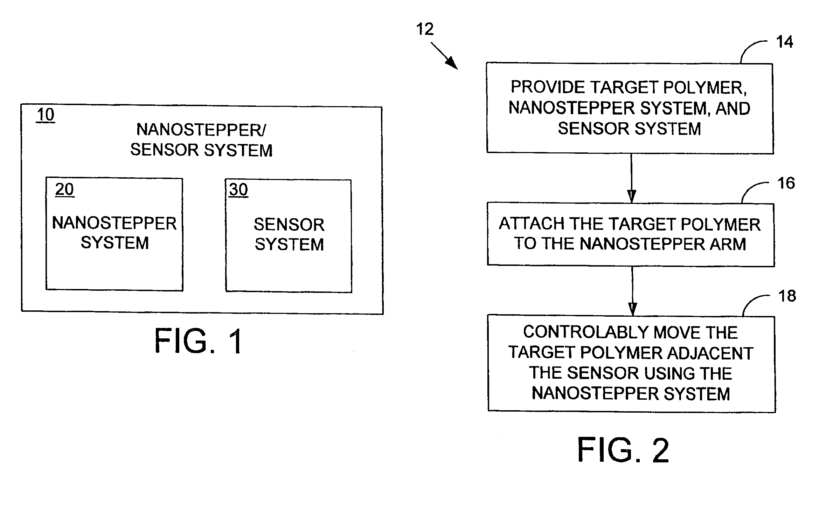

FIG. 1 illustrates a representative embodiment of a nanostepper/sensor system 10 that can be used to analyze, controllably and reversibly, target polymers. The target polymers can include, but are not limited to, biopolymers, polypeptides (e.g., proteins and portions thereof), polynucleotides (*e.g*., DNA, RNA, PNA, and portions thereof), synthetic polymers (e.g., copolymer and block polymers), and the like. The nanostepper/sensor system 10 includes, but is not limited to, a nanostepper system 20 and a sensor system 30. The nanostepper system 20 and the sensor system 30 are operative to position target polymers in relation to a sensor in the sensor system 30 to measure one or more characteristics of the target molecule. The position of the target polymer and movement (e.g., rate and step size) thereof are controlled by the nanostepper system 20. The movement can be performed in a forward or backward manner upon the target polymer. In addition, the positioning of the target polymer in relation to the sensor can be reproduced and repeated, which enables accurate and precise measurements to be performed on the same molecule.

FIG. 2 is a flow diagram illustrating a representative process 12 for using the nanostepper/sensor system 10. As shown in FIG. 2, the functionality (or method) may be construed as beginning at block 14, where a target polymer, the nanostepper system 20, and the sensor system 30, are provided. In block 16, the target polymer is attached (e.g., covalently, ionicly, biochemically, mechanically, electronically, magnetically, and the like) to a nanostepper arm. In block 18, the nanostepper system 20 moves the target polymer relative to the sensor in a controlled manner. As a result, the sensor system 30 is operative to measure one or more characteristics of the target polymer.

In general, the nanostepper system includes an x-/y-direction moving structure having one or more nanostepper arms attached therewith. The x-/y-direction moving structure is operative to move independently in the x- and y-directions in a controllable and reproducible manner. The nanostepper system can also include a z-direction moving structure that is operative to move in the z-direction in a controllable and reproducible manner and can move independently from the x-/y-direction moving structure. Using the x-/y-direction moving structure and/or the z-direction moving structure, the nanostepper system can produce a mechanical force to move the target polymer in relation to the sensor. In other words, the nanostepper system is capable of moving the target polymer in three dimensions. In another embodiment, the nanostepper system can use an electrical and/or magnetic force in conjunction with the mechanical force to move the target polymer in relation to the sensor.

A portion of the nanostepper arm can include one or more chemicals, biochemicals, magnetic structures, conductive structures, and combinations thereof, to interact with the target polymer. The interaction between the nanostepper arm and the target polymer should be sufficiently robust to withstand the forces exerted while moving the target polymer in relation to the sensor. In one embodiment, the strength of the interaction between the nanostepper arm and the target polymer should be at least as strong as the bonds in the backbone (e.g., sugar backbone of polynucleotides and polypeptides) of the target polymer. The target polymer can be disposed onto the nanostepper arm using one or more interactions such as, but not limited to, covalent bonds, bio-conjugation binding (e.g., biotin-streptavidin interactions), hybridization interactions with a portion of the target polymer (e.g., using DNA, RNA, and PNA), magnetic interactions (e.g., the target polymer includes a magnetic structure), zinc-finger proteins, and combinations thereof.

The nanostepper is operative to position the target polymer and move (e.g., pull) it past (e.g., adjacent and in sufficiently close proximity) a sensor in a controllable, repeatable, and reversible manner so that the sensor can measure one or more characteristics of the molecules of the target polymer.

In general, the x-direction is defined as an axis through a structure in which a nanopore is formed of the sensor system 30. The direction parallel to the plane of the sensor system 30 will be referred to as the y-direction. The direction orthogonal to both the x- and y-directions will be referred to as the z-direction. In an embodiment, the x-direction is an axis that substantially passes through the nanopore, while in another embodiment, the x-direction is an axis that is within an angle of about 60 to 75 degrees perpendicular to the axis of the sensor system 30.

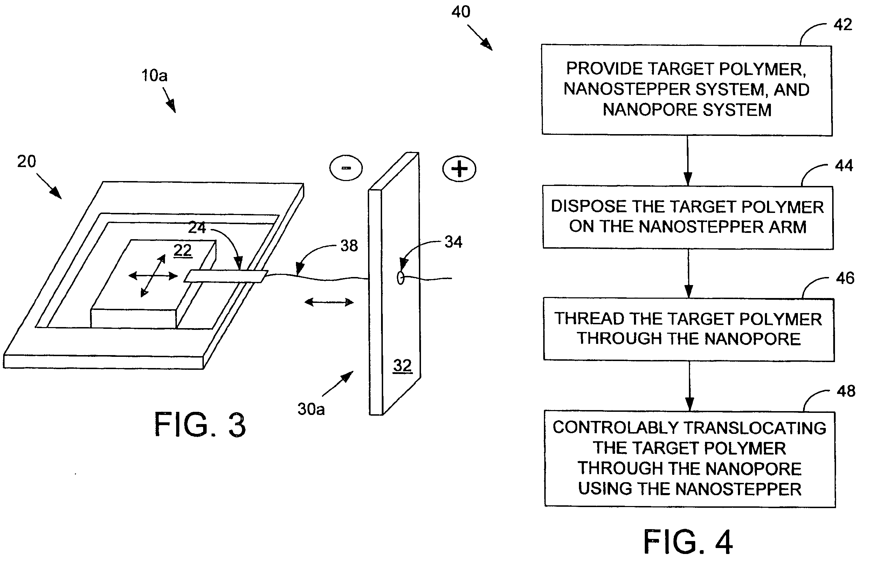

As illustrated in FIG. 3, the x-direction is in the same plane as the sensor so that movement in the x-direction moves the target polymer forward and backward past the sensor. The y-direction is also in the same plane as the sensor but movement in the y-direction moves the target polymer to the left and right of the sensor. In another embodiment, the nanostepper is operative to move in the z-direction, which is in a plane parallel the sensor. Movement in the z-direction moves the target polymer above and below the sensor.

The range of motion of the nanostepper system depends on the design specifications of the type of nanostepper used in a particular application. In typical embodiments, the range of motion of the nanostepper is at least about ±0.5 µmeters (e.g., at least about ±1 µmeters, or at least about ±2.5 µmeters, or at least about ±5 µmeters). In typical embodiments, the range of motion of the nanostepper system is up to about ±60 µmeters (e.g., about ±50 µmeters, about ±40 µmeters, about ±30 µmeters, or about ±20 µmeters, or about ±10 µmeters). In other embodiments, the nanostepper system may have a range of motion smaller or larger than indicated above. The range of motion described in this paragraph is measured from a center position of the nanostepper system and given as "plus or minus" a given value; it should be understood that the full range of motion is thus twice the given value (e.g., ±35 µmeters provides a full range of motion of 70 µmeters).

In addition, the nanostepper can move in a step size from about 1 to 4000 Angstroms at a stepping speed of about 1 to 1,000,000 steps per second. If the target polymer is secured at two points, one point being the nanostepper arm, the nanostepper can exert a force of about 1 nanoNewton to 500 µNewtons on the target polymer, thereby being capable of stretching the target polymer into a substantially linear configuration.

Additional details regarding the nanostepper system are described in detail in "A High-Performance Dipole Surface Drive for Large Travel and Force," Storrs Hoen, Qing Bai, Jonah A. Harley, *et al.;* Transducers 2003 12th International Conference on Solid State Sensors, Actuators, and Microsystems, Boston, June 8-12, 2003; "Electrostatic Surface Drives: theoretical considerations and fabrication," Storrs Hoen, Paul Merchant, Gladys Koke, Judy Williams, Hewlett Packard Laboratories; Transducers 1997, 1997 International Conference on Solid-State Sensors and Actuators, Chicago, June 16-19, 1997; U.S. Patent No. 5,986,381; U.S. Patent No. 6,657,359; U.S. Patent No. 6,695,297; U.S. Patent No. 6,541,892; U.S. Patent No. 6,210,896; U.S. Patent Application No. 20020110818, and U.S. Patent Application No. 20020039737, each of which is incorporated herein by reference.

In general, the sensor system is operative to sense characteristics of the target polymer as the target polymer is moved relative to the sensor. For example, the sensor system can determine the sequence of the target polymer by sensing each molecule as it passes in close proximity of the sensor (e.g., determining the nucleotide sequence of a polynucleotide). The sensor system can include systems such as, but not limited to, a nanopore system using various sensing techniques. The kinds and types of sensors depend upon the type of sensor system being used and the measurements being conducted. An illustrative example of a sensor includes a system operative to detect electrical characteristics of the molecules of the target polymer. For example, the amplitude and/or duration of individual conductance or electron tunneling current changes corresponding to the molecules of the target polymer can be sensed as it moves past an aperture having an appropriate electronic sensing system interfaced therewith.

In one embodiment, the sensor system 30 is a nanopore system to detect/analyze/monitor characteristics of the target polymers such as, but not limited to, polynucleotides, polypeptides, combinations thereof, and specific regions thereof. For example, nanopore sequencing of polynucleotides and/or polypeptides (but hereinafter polynucleotides for clarity) has been described (U.S. Pat. No. 5,795,782 to Church *et al.*; U.S. Pat. No. 6,015,714 to Baldarelli *et al.,* the teachings of which are both incorporated herein by reference).

In general, nanopore sequencing involves the use of two separate pools of a medium and an interface between the pools. The interface between the pools is capable of interacting sequentially with the individual monomer residues of a polynucleotide present in one of the pools. The nanostepper system can be located on either side of the interface and in some embodiments a nanostepper system can be located on both sides of the interface.

Interface dependent measurements are continued over time, as individual monomer residues of the polynucleotide interact sequentially with the interface, yielding data suitable to infer a monomer-dependent characteristic of the polynucleotide. The monomer-dependent characterization achieved by nanopore sequencing may include identifying physical characteristics such as, but not limited to, the number and composition of monomers that make up each individual polynucleotide, in sequential order.

In this embodiment, the term "sequencing" means determining the sequential order of nucleotides in a polynucleotide molecule. Sequencing as used herein includes in the scope of its definition, determining the nucleotide sequence of a polynucleotide in a *de novo* manner in which the sequence was previously unknown. Sequencing as used herein also includes in the scope of its definition, determining the nucleotide sequence of a polynucleotide wherein the sequence was previously known. Sequencing polynucleotides, the sequences of which were previously known, may be used to identify a polynucleotide, to confirm a polynucleotide, or to search for polymorphisms and genetic mutations.

FIG. 3 illustrates a representative embodiment of a nanostepper/sensor system 10a. The nanostepper/sensor system 10a includes, but is not limited to, a nanostepper system 20 and a nanopore system 30a including a nanopore aperture 34. The nanostepper system 20 includes, but is not limited to, an x-/y-direction moving structure 22 and a nanostepper arm 24 attached thereto. As described above, the x-/y-direction moving structure 22 can move in the x-direction, which is in the same plane as the sensor (not shown, but adjacent the nanopore aperture 34) and nanopore aperture 34 so that movement in the x-direction moves the target polymer 38 forward and backward past the sensor. The y-direction is also in the same plane as the sensor but movement in the y-direction moves the target polymer 38 to the left and right of the sensor. Additional details about the nanostepper system 20 are described above.

The nanopore system 30a can include, but is not limited to, a structure 32 that separates two independent adjacent pools of a medium. The two adjacent pools are located on the cis side and the trans side of the structure 32. The structure 32 includes, but is not limited to, at least one nanopore aperture 34 so dimensioned as to allow sequential monomer-by-monomer translocation (i.e., passage) from one pool to another of only one polynucleotide at a time, and detection components that can be used to perform measurements of the target polynucleotide 38.

Exemplary detection components for nanopore systems 30a have been described in WO 00/79257 and can include, but are not limited to, electrodes directly associated with the structure 32 at or near the nanopore aperture 34, and electrodes placed within the cis and trans pools. The electrodes may be capable of, but not limited to, detecting ionic current differences across the two pools or electron tunneling currents across the pore aperture.

In general, the sensing is performed as the target polynucleotide 38 translocates through or passes sufficiently close to the nanopore aperture 34. Measurements (e.g., ionic flow measurements, including measuring duration or amplitude of ionic flow blockage) can be taken by a nanopore detection system as each of the nucleotide monomers of the target polynucleotide 38 passes through or sufficiently close to the nanopore aperture 34. The measurements can be used to identify the sequence and/or length of the target polynucleotide 38.

The structure 32 can be made of materials such as, but not limited to, silicon nitride, silicon oxide, mica, polyimide, silicon, and combinations thereof. The structure 32 can include, but is not limited to, detection electrodes and detection integrated circuitry. The structure 32 can include one or more nanopore apertures 34. The nanopore aperture 34 can be dimensioned so that only a single stranded polynucleotide can translocate through the nanopore aperture 34 at a time or that a double or single stranded polynucletide can translocate through the nanopore aperture 34. The nanopore aperture 34 can have a diameter of about 3 to 5 nanometers (for analysis of single or double stranded polynucleotides), and from about 2 to 4 nanometers (for analysis of single stranded polynucleotides). Depending upon the method of detection used, the size of the nanopore aperture 34 may be significantly larger than the radial dimension of polynucleotide. In another embodiment, the nanopore aperture can be dimensioned to allow a polypeptide to pass through the nanopore aperture.

The nanopore detection system includes, but is not limited to: electronic equipment capable of measuring characteristics of the polynucleotide as it interacts with the nanopore aperture; a computer system capable of controlling the measurement of the characteristics and storing the corresponding data; control equipment capable of controlling the conditions of the nanopore system; and components that are included in the nanopore system that are used to perform the measurements, as described below.

The nanopore detection system can measure characteristics such as, but not limited to, the amplitude or duration of individual conductance or electron tunneling current changes across the nanopore aperture 34. Such changes can identify the monomers in sequence, as each monomer has a characteristic conductance change signature. For instance, the volume, shape, or charges on each monomer can affect conductance in a characteristic way. Alternatively, the number of nucleotides in the target polynucleotide 38 (also a measure of size) can be estimated as a function of the number of nucleotide-dependent conductance changes for a given nucleic acid traversing the nanopore aperture 34. The number of nucleotides may not correspond exactly to the number of conductance changes because there may be more than one conductance level change as each nucleotide of the nucleic acid passes sequentially through the nanopore aperture 34. However, there is proportional relationship between the two values that can be determined by preparing a standard with a polynucleotide having a known sequence.

The medium disposed in the pools on either side of the substrate 32 may be any fluid that permits adequate polynucleotide mobility for substrate 32 interaction. Typically, the medium is a liquid, usually aqueous solutions or other liquids or solutions in which the polynucleotides can be distributed. When an electrically conductive medium is used, it can be any medium which is able to carry electrical current. Such solutions generally contain ions as the current-conducting agents (e.g., sodium, potassium, chloride, calcium, cesium, barium, sulfate, or phosphate). Conductance across the nanopore aperture 34 can be determined by measuring the flow of current across the nanopore aperture 34 via the conducting medium. A voltage difference can be imposed across the barrier between the pools using appropriate electronic equipment. Alternatively, an electrochemical gradient may be established by a difference in the ionic composition of the two pools of medium, either with different ions in each pool, or different concentrations of at least one of the ions in the solutions or media of the pools. Conductance changes are measured by the nanopore detection system and are indicative of monomer-dependent characteristics.

The nanostepper moves the target polynucleotide 38 in relation to the nanopore aperture 34 causes individual nucleotides interact sequentially with the nanopore aperture 34 to induce a change in the conductance of the nanopore aperture 34.

FIG. 4 is a flow diagram illustrating of a representative process 40 for using the nanostepper/sensor system 10a. As shown in FIG. 4, the functionality (or method) may be construed as beginning at block 42, where the target polymer 38, the nanostepper system 20, and the nanopore system 30a are provided. In block 44, the target polymer 38 is disposed (e.g., covalently, ionicly, biochemically, mechanically, electronically, magnetically, and the like) to a nanostepper arm 24 of the nanostepper system 20. In block 46, the target polymer 38 is threaded through the nanopore aperture 34. The target polymer 38 can be threaded through the nanopore aperture 34 using forces (e.g., fields) such as, but not limited to, electronic, electrophoretic, magnetic, and combinations thereof. For example, the target polymer 38 can be drawn to the nanopore aperture 34 using a voltage applied to the nanopore. In block 48, the nanostepper system 20 moves the target polymer 38 relative to the sensor in a controlled manner. As a result, the sensor system 30 is operative to measure one or more characteristics of the target polymer 38.

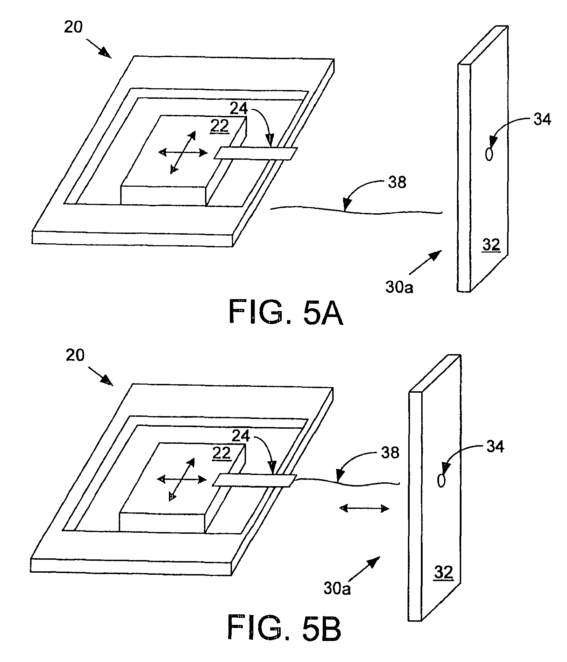

FIGS. 5A through 5D illustrate a representative process for using the nanostepper/sensor system. FIG. 5A illustrates the nanostepper system 20, as described above, the nanopore system 30a, as described above, and the target polymer 38. In FIG. 5B the target polymer 38 (e.g., polynucleotide) is disposed onto the nanostepper arm 24 using one or more interactions described above.

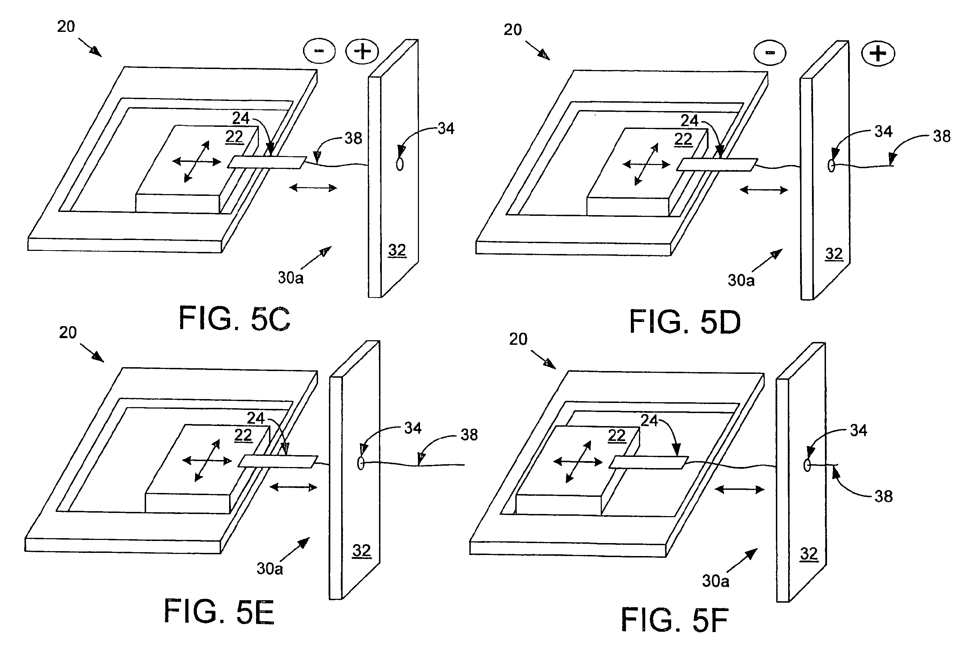

FIG. 5C illustrates a method of threading the target polynucleotide through the nanopore. A first voltage is applied between the nanostepper arm and the cis side of the nanopore structure 32. This creates an electrical force that attracts the unattached end of the target polymer 38 towards the cis side of the nanopore aperture 34, bringing it in close proximity to the nanopore aperture 34.

In FIG. 5D, a second voltage is placed across the nanopore structure 32 so that the target polymer 38 translocates through of the nanopore aperture 34 and into the trans side of the nanopore aperture 34. Once the target polymer 38 is threaded through the nanopore aperture 34, the voltage gradient may be turned off. In this embodiment, the positioning and movement of the target polymer 38 is mechanical and driven by the nanostepper. In another embodiment, the voltage gradient may be left on. The attractive force used to thread the target polymer through the nanopore can be used to apply tension and straighten the target polymer 38. In another embodiment, the first voltage and the second voltage can be used simultaneously.

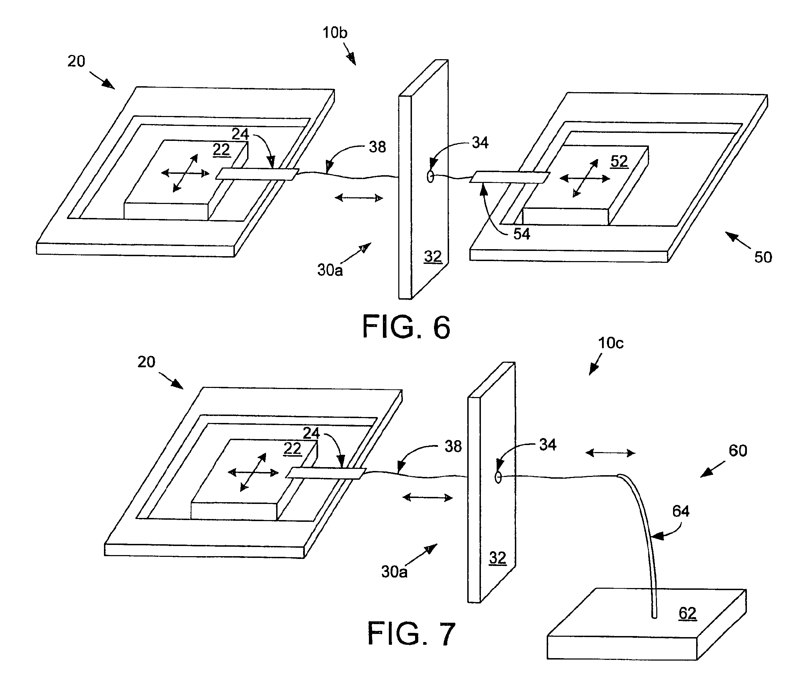

In another embodiment, a magnetic structure can be disposed on the target polymer 38 at the end opposite the attachment of the nanostepper arm. Then by applying a magnetic field to the nanostepper/sensor system 10a, the target polymer 38 is straightened as the x-/y-direction moving structure 22 is moved in the direction opposite the magnetic field. In still other embodiments, the target polymer 38 at the end opposite the attachment of the nanostepper arm 24 can be attached to another structure such as those described in FIGS. 6 and 7.

FIGS. 5E and 5F illustrate the translocation of the target polymer 38 into and out of the nanopore aperture 34 by the nanostepper system 20. A signal corresponding to the translocation of the target polymer 38 through the nanopore aperture 34 is monitored by the nanopore detection system. After the analysis is complete, the target polymer 38 can be released (e.g., electrical repulsion, pH change, salt concentration change, and the like) from the nanostepper arm 24.

FIG. 6 illustrates a representative embodiment of a nanostepper/sensor system 10b. The nanostepper/sensor system 10b includes, but is not limited to, the nanostepper system 20, the nanopore system 30a, and a second nanostepper system 50. The nanostepper system 20 and the second nanostepper system 50 are disposed on the cis and trans side of the nanopore system 30a, respectively. The second nanostepper system 50 includes an x-/y-direction moving structure 52 having a second nanostepper arm 54 disposed thereon. The opposite ends of the target polymer 38 are disposed on each nanostepper arm 24 and 54. The target polymer 38 is disposed on the second nanostepper arm 54 (in a manner as described above) after being threaded through the nanopore aperture 34, or vice versa. The two nanostepper systems 20 and 50 can be used to move the target polymer 38 back and forth through the nanopore aperture 34. In addition, the nanostepper systems 20 and 50 can be used to substantially straighten the target polymer 38. The two nanostepper systems can be operated in a coordinated fashion to accurately control the position and tension of the target polymer.

FIG. 7 illustrates a representative embodiment of a nanostepper/sensor system 10c. The nanostepper/sensor system 10c includes, but is not limited to, the nanostepper system 20, the nanopore system 30a, and a flexure system 60. The nanostepper system 20 and flexure system 60 are disposed on the cis and trans side of the nanopore system 30a, respectively. The flexure system 60 includes, but is not limited to, a flexure 64 attached to a flexure base 62. The flexure 64 is mechanically flexible and can provide a counter-force to the x-/y-direction moving structure 52, which can be used to straighten the target polymer 38. The target polymer 38 can be disposed onto the flexure 64 through the interactions such as those described above and which include covalent, ionic, biochemical, mechanical, electrical, and magnetic interactions.

One end of the target polymer 38 is disposed on the nanostepper arm 24, while the opposite end of the target polymer 38 is disposed on the flexure 64. The target polymer 38 is disposed on the flexure 64 after being threaded through the nanopore aperture 34, or vice versa. The nanostepper systems 20 and the flexure 64 can be used to move the target polymer 38 back and forth through the nanopore aperture 34. In addition, the nanostepper systems 20 and the flexure 64 can be used to substantially straighten the target polymer 38.

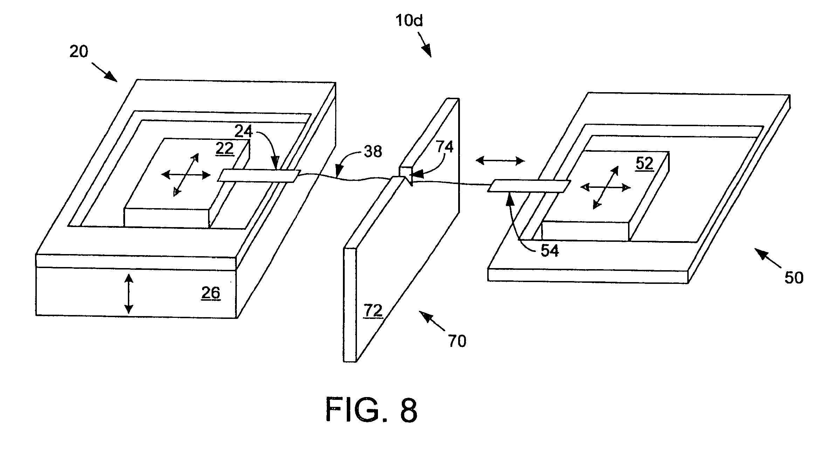

FIG. 8 illustrates a representative embodiment of a nanostepper/sensor system 10d. The nanostepper/sensor system 10d includes, but is not limited to, the nanostepper system 20, a notch system 70, and a second nanostepper system 50. The notch system 70 includes a structure 72 having a notch (slit) 74 disposed at the top of the structure 72. The term notch can also include slits and other indentations in the structure 72 that accomplish the same result. In addition to the components described above, the nanostepper system 20 includes a z-direction moving structure 26 which is operative to move in the z-direction in a controllable and reproducible manner independently from the x-/y-direction moving structure. There is only a single fluid in systems using a notch 74. The notch 74 can have a shape that narrows to about a 1 nanometer sized apex. For example, the notch 74 can be in the shape of a triangle. A nanopore detection system is disposed adjacent the notch 74 and operates and senses the target polymer 38 in the same manner as in the nanopore system. The nanostepper system 20 and the second nanostepper system 50 are disposed on the cis and trans side of the notch system 70, respectively.

The opposite ends of the target polymer 38 are disposed on each nanostepper arm 24 and 54. The target polymer 38 can be disposed on each nanostepper arm 24 and 54 prior to being threaded through the notch 74. Subsequently, the target polymer 38 can be moved into the notch 74 using a combination of the x-/y-direction moving structures 22 and 52 and the z-direction moving structure 26. In other words, the nanostepper system 20 can be moved up and down with the z-direction moving structure 26 to position the target polymer 38 sufficiently within the notch 74. Thereafter, the two x-/y-direction moving structures 22 and 52 can be used to move the target polymer 38 back and forth through the notch aperture 74. In addition, the nanostepper systems 20 and 50 can be used to substantially straighten the target polymer 38.

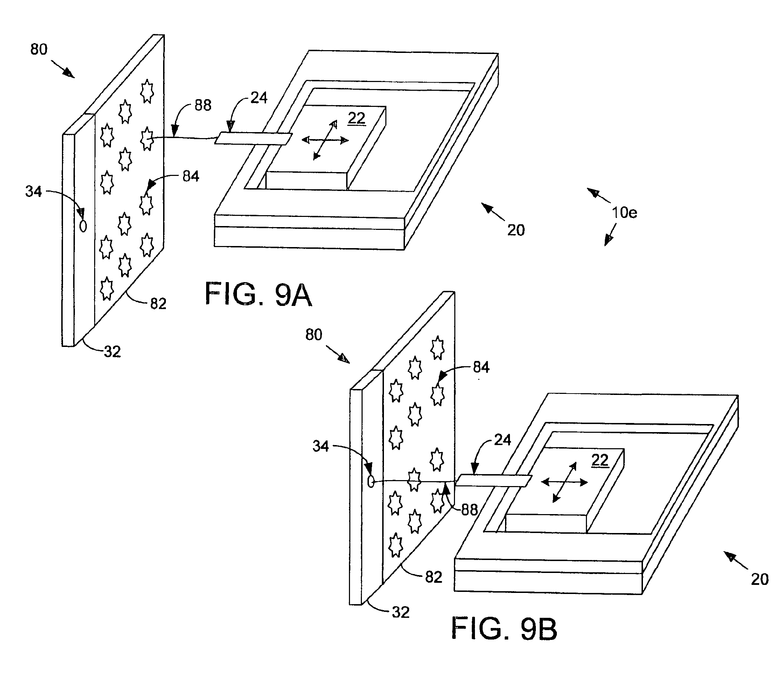

FIGS. 9A and 9B illustrate a representative embodiment of a nanostepper/sensor system 10e. The nanostepper/sensor system 10e includes, but is not limited to, the nanostepper system 20 and a nanopore/array system 80. In addition to the components described above, the nanostepper system 20 includes a z-direction moving structure 26, which is operative to move in the z-direction in a controllable and reproducible manner and can move independently from the x-/y-direction moving structure. The nanopore/array system 80 can include, but is not limited to, the structure 32 having the nanopore aperture 34 thereon (analogous to the nanopore system 30a) and an array 82 having a plurality of discrete areas (spots) 84 thereon. The array 82 can include, but is not limited to, arrays and microarrays such as those known by one skilled in the art. The discrete areas can be fabricated to interact with one or more target polymers. The array 82 can be disposed adjacent the nanopore system or disposed a distance away from the nanopore system such that the nanostepper system 20 can move between the array 82 and the nanopore system. The nanostepper system 20 is capable of long-range movement over many microns (e.g., about 100 microns) to pick up target polymers from the array area 82, move them back to the vicinity of the nanopore aperture 34, and then perform accurate and repeatable motion on the scale of a few Angstroms during the detection process, which is distinct from the use of piezoelectric actuators.

Initially, the nanostepper system 20 positions itself substantially in-line with a discrete area having the target polymer 88 disposed thereon. The target polymer 88 interacts with the nanostepper arm 24 at the end opposite the array 82. Then the target polymer 88 is released from the array 82. Subsequently, the nanostepper system 20 moves the nanostepper arm 24 to be substantially in-line with the nanopore aperture. Thereafter, the target polymer 88 can be translocated through the nanoaperture in a manner as described herein using the nanostepper system 20.

It should be emphasized that many variations and modifications may be made to the above-described embodiments. All such modifications and variations are intended to be included herein within the scope of this disclosure and protected by the following claims.

## Claims

1. A method for analyzing a polymer, comprising:
translocating a target polymer (38) through a nanopore aperture (34) in a controllable, repeatable, and reversible manner using a first x-/y-direction moving structure (22), wherein the first x-/y-direction moving structure (22) is operative to position the target polymer (38) substantially in-line with the nanopore aperture (34), wherein the first x-/y-direction moving structure (22) is operative to move independently in the x- and y-directions, and wherein the x-direction is defined as an axis through a structure in which the nanopore aperture (34) is formed; and
monitoring a signal corresponding to the movement of the target polymer (38) with respect to the nanopore aperture (34) as a function of the movement of the first x-/y-direction moving structure (22).

2. The method of claim 1, further comprising:
providing a nanopore system (30a) including the structure having the nanopore aperture (34);
providing a first nanostepper system (20) having the first x-/y-direction moving structure (22) and a first nanostepper arm (24), wherein the first x-/y-direction moving structure (22) is operative to move the first nanostepper arm (24) independently in the x- and y-directions, wherein the y-direction is in a plane perpendicular to the nanopore aperture (34) and moves the first nanostepper arm (24) to the right and left of the nanopore aperture (34);
immobilizing the target polymer (38) on the first nanostepper arm (24), wherein the target polymer (38) can be disposed adjacent the nanopore aperture (34) such that the first nanostepper arm (24) is substantially inline with the nanopore aperture (34);
threading the target polymer (38) through the nanopore aperture (34);
translocating the target polymer (38) through the nanopore aperture (34) in a controllable, repeatable, and reversible manner using the first nanostepper system (20); and
monitoring a signal corresponding to the movement of the target polymer (38) through the nanopore aperture (34).

3. The method of claim 1 or 2, further comprising:
moving the x-/y-direction moving structure (22), wherein the movement causes the target polymer (38) to translocate through the nanopore aperture (34).

4. A system, comprising:
a nanopore system (30a) including a structure having a nanopore aperture (34); and
a first nanostepper system (20) having an x-/y-direction moving structure (22) and a first nanostepper arm (24) positioned adjacent the structure (32), wherein the first nanostepper arm (24) is adapted to interact with a target polymer (38), wherein the x-/y-direction moving structure (22) is operative to position the first nanostepper arm 24 having the target polymer (38) disposed thereon substantially inline with the nanopore aperture (34), and wherein the x-/y-direction moving structure (22) is operative to controllably translocate the target polymer (38) through the nanopore aperture (34).

5. The system of claim 4, wherein the first nanostepper system (20) can repetitively and controllably translocate the target polymer (38) through the nanopore aperture (34) using the x-/y-direction moving structure (22).

6. A method for analyzing a polymer, comprising:
moving a target polymer (38) adjacent a sensor in a controllable, repeatable, and reversible manner using a nanostepper system (20), wherein the nanostepper system (20) is operative to position the target polymer (38) substantially near the sensor, wherein the nanostepper system (20) is operative to move independently in the x- and y-directions, wherein the x-direction is in the same plane as the sensor and the y-direction moves the target polymer (38) to the left and right of the sensor; and
monitoring the signal corresponding to the movement of the target polymer (38) with respect to the sensor as a function of the movement of the nanostepper system (20).

7. A system, comprising:
a sensor system (30) including a sensor; and
a nanostepper system (20) having an x-/y-direction moving structure (22) and a nanostepper arm (24) positioned adjacent the sensor, wherein the nanostepper arm (24) is adapted to interact with a target polymer (38), wherein the x-/y-direction moving structure (22) is operative to position the nanostepper arm (24) having the target polymer (38) disposed thereon substantially adjacent the sensor, wherein the x-/y-direction moving structure (22) is operative to controllably and reversibly move the target polymer (38) near the sensor such that the sensor senses the target polymer (38).

8. The system of claim 7, wherein the nanostepper system (20) is operative to move ±60 µmeters in the x-direction and wherein the nanostepper system (20) is operative to move ±60 µmeters in the y-direction.

9. The system of claim 7, wherein the nanostepper system (20) is operative to move in a step size from about 1 to 4000 Angstroms at a stepping speed of about 1 to 1,000,000 steps per second.

10. The system of claim 7, wherein the nanostepper system (20) is operative to stretch the target polymer (38) with a force of about 1 nanoNewton to 500 µNewtons.
